(19) Europäisches Patentamt
European Patent Office
Office européen des brevets

(11) **EP 2 779 988 B1**

(12) **EUROPÄISCHE PATENTSCHRIFT**

(45) Veröffentlichungstag und Bekanntmachung des Hinweises auf die Patenterteilung:
**23.11.2016 Patentblatt 2016/47**

(21) Anmeldenummer: **12769082.4**

(22) Anmeldetag: **27.09.2012**

(51) Int Cl.:
*A61K 8/81* (2006.01)   *A61Q 5/06* (2006.01)

(86) Internationale Anmeldenummer:
**PCT/EP2012/069085**

(87) Internationale Veröffentlichungsnummer:
**WO 2013/072118 (23.05.2013 Gazette 2013/21)**

(54) **MITTEL ZUR TEMPORÄREN VERFORMUNG KERATINISCHER FASERN AUF GRUNDLAGE EINER KOMBINATION SPEZIFISCHER FILMBILDENDER POLYMERE**

COMPOSITION FOR TEMPORARY DEFORMATION OF KERATINIC FIBRES, BASED ON A COMBINATION OF SPECIFIC FILM-FORMING POLYMERS

PRODUIT SERVANT À LA MISE EN FORME TEMPORAIRE DE FIBRES KÉRATINIQUES, À BASE D'UNE COMBINAISON DE POLYMÈRES FILMOGÈNES SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2011 DE 102011086559**

(43) Veröffentlichungstag der Anmeldung:
**24.09.2014 Patentblatt 2014/39**

(73) Patentinhaber: **Henkel AG & Co. KGaA**
**40589 Düsseldorf (DE)**

(72) Erfinder:
• **METTEN, Diana**
  **22393 Hamburg (DE)**
• **RICHTERS, Bernd**
  **21129 Hamburg (DE)**
• **THOMS, Johanna**
  **22299 Hamburg (DE)**

(56) Entgegenhaltungen:
**WO-A1-2011/057882    US-A1- 2002 028 187**

Bemerkungen:
Die Akte enthält technische Angaben, die nach dem Eingang der Anmeldung eingereicht wurden und die nicht in dieser Patentschrift enthalten sind.

EP 2 779 988 B1

**Beschreibung**

Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinse-off Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen.

Die internationale Patentanmeldung WO 2011/057882 A1 beschreibt ein Tetrapolymer mit guten kosmetischen Eigenschaften, insbesondere im Bereich der Haarpflege und Haarverformung. Dieses Tetrapolymer wird durch Polymerisation der Monomere n-Butylmethacrylat, Methacrylsäure, Ethylacrylat und Ethylmethacrylat erhalten. Die WO 2011/057882 A1 beschreibt weiterhin die Kombination dieses speziellen Tetrapolymers mit weiteren filmbildenden Polymeren in Mitteln zur Verformung keratinischer Fasern. Zur Gruppe dieser weiteren filmbildenden Polymere zählt u.a. auch ein als Amphomer® bezeichnetes Terpolmyer auf Grundlage der Monomere n-tert-Octylacrylamid, Acrylsäure und tert.-Butylaminoethylmethacrylsäure. Entsprechende Polymergemische erwiesen sich bei Einsatz in Mitteln zur temporären Verformung keratinischer Fasern jedoch als verbesserungswürdig.

Die Aufgabe der vorliegenden Erfindung war es daher, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen.

Diese technische Aufgabe konnte durch eine Kombination der beiden vorgenannten Polymere in einem spezifischen Gewichtsverhältnis gelöst werden.

Ein erster Gegenstand der vorliegenden Anmeldung ist daher ein kosmetisches Mittel, enthaltend in einem kosmetisch akzeptablen Träger

    a) mindestens ein Copolymer A aus den Monomeren

        a1) n-Butylmethacrylat
        a2) Methacrylsäure
        a3) Ethylacrylat
        a4) Ethylmethacrylat

sowie gegebenenfalls weiteren Monomeren

b) mindestens ein von Copolymer A unterschiedliches Copolymer B aus den Monomeren

    b1) N-tert-Octylacrylamid
    b2) Acrylsäure
    b3) tert.-Butylaminoethylmethacrylsäure

sowie gegebenenfalls weiteren Monomeren, dadurch gekennzeichnet, dass das Verhältnis der Gewichtsanteile von Copolymer A und Copolymer B in dem Mittel zwischen 2:3 und 3:2 beträgt.

Die erfindungsgemäßen Mittel zeichnen sich von kosmetischen Mitteln mit einem abweichenden Gewichtsverhältnis der Copolymere A und B neben den oben genannten Vorteilen insbesondere auch durch einen verbesserten Haltegrad aus.

Die erfindungsgemäß eingesetzten Copolymere A werden durch Polymerisation der Monomere n-Butylmethacrylat, Methacrylsäure, Ethylacrylat und Ethylmethacrylat erhalten. Bevorzugte Copolymere A bestehen zu mindestens 90 Gew.-%, vorzugsweise zu mindestens 95 Gew.-% und insbesondere zu mindestens 97 Gew.-% aus den Monomeren n-Butylmethacrylat, Methacrylsäure, Ethylacrylat und Ethylmethacrylat. Besonders bevorzugt werden Copolymere A, die ausschließlich aus den Monomeren n-Butylmethacrylat, Methacrylsäure, Ethylacrylat und Ethylmethacrylat erhalten wurden.

In einer bevorzugten Ausführungsform weisen die Copolymere A 35 bis 65 Gew.-% n-Butylmethacrylat, 10 bis 30 Gew.-% Methacrylsäure, 5 bis 15 Gew.-% Ethylacrylat und 10 bis 35 Gew.-% Ethylmethacrylat auf, wobei Copolymere A mit einem Gewichtsanteil der Monomere von 38 bis 60 Gew.-% n-Butylmethacrylat, 15 bis 25 Gew.-% Methacrylsäure, 8 bis 15 Gew.-% Ethylacrylat und 15 bis 25 Gew.-% Ethylmethacrylat besonders bevorzugt sind. Ganz besonders bevorzugte Copolymere A weisen einen Gewichtsanteil der Monomere von 44 bis 56 Gew.-% n-Butylmethacrylat, 17 bis 22 Gew.-% Methacrylsäure, 9 bis 15 Gew.-% Ethylacrylat und 15 bis 25 Gew.-% Ethylmethacrylat auf.

Das Gewichtsverhältnis der Monomere Ethylmethacrylat und Ethylacrylat in den Copolymeren A beträgt vorzugsweise 4:1 bis 1:1, bevorzugt 2:1 bis 1:1 und insbesondere 2:1 bis 1,3:1.

Bevorzugte Copolymere A bestehen zu mindestens 90 Gew.-%, vorzugsweise zu mindestens 95 Gew.-% und insbesondere zu mindestens 97 Gew.-% aus den Monomeren n-Butylmethacrylat, Methacrylsäure, Ethylacrylat und Ethylmethacrylat.

Die Copolymere A weisen vorzugsweise ein Molekulargewicht von etwa 100kDa auf. Die Glasübergangstemperatur beträgt vorzugsweise zwischen 80 und 120°C. Die Partikelgröße der Polymerpartikel (X-50) liegt bevorzugt im Bereich zwischen 50 und 500 $\mu$m, vorzugsweise zwischen 200 und 500 $\mu$m.

Die zuvor beschriebenen Copolymere A werden beispielsweise unter der Bezeichnung Tilamar® Fix A1000 (INCI: Acrylates Copolymer; CAS Nummer: 1070166-98-1) von der Firma DSM vertrieben.

Bevorzugte erfindungsgemäße Mittel sind dadurch gekennzeichnet, dass der Gewichtsanteil des Copolymers A am Gesamtgewicht des kosmetischen Mittels zwischen 0,5 und 10 Gew.-%, vorzugsweise zwischen 1,0 und 8,0 Gew.-% und insbesondere zwischen 2,0 und 8,0 Gew.-% beträgt.

Die erfindungsgemäß eingesetzten Copolymere B werden durch Polymerisation der Monomere N-tert-Octylacrylamid, Acrylsäure und tert.-Butylaminoethylmethacrylsäure erhalten. Bevorzugte Copolymere A bestehen zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylsäure. Besonders bevorzugte Copolymere B wurden ausschließlich aus den Monomeren N-tert-Octylacrylamid, Acrylsäure und tert.-Butylaminoethylmethacrylsäure erhalten.

Die zuvor beschriebenen Copolymere B werden beispielsweise unter der Bezeichnung Amphomer® (INCI-Bezeichnung: Octylacrylamide / Acrylates / Butylaminoethyl Methacrylate Copolymer) von der Firma National Starch vertrieben.

Bevorzugte erfindungsgemäße Mittel sind dadurch gekennzeichnet, dass der Gewichtsanteil des Copolymers B am Gesamtgewicht des kosmetischen Mittels zwischen 0,5 und 10 Gew.-%, vorzugsweise zwischen 1,0 und 8,0 Gew.-% und insbesondere zwischen 2,0 und 8,0 Gew.-% beträgt.

Die Copolymere A und/oder B werden in dem kosmetischen Mitteln vorzugsweise in teilneutralisierter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten daher 2-Amino-2-methylpropanol. Das 2-Amino-2-methylpropanol wird in den erfindungsgemäßen Mitteln vorzugsweise in einer Menge eingesetzt, welche die zur Neutralisation der Copolymere A und B benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation der Copolymere A und B benötigten Menge. In einer bevorzugten Ausführungsform beträgt der Gewichtsanteil des 2-Amino-2-methylpropanols am Gesamtgewicht des kosmetischen Mittels 0,1 bis 4,0 Gew.-%, bevorzugt 0,2 bis 3,0 Gew.% und insbesondere 0,5 bis 2,0 Gew.-%.

Die erfindungsgemäßen kosmetischen Mittel können neben den beiden zuvor beschriebenen Copolymeren A und B weitere filmbildende und/oder festigende Polymere enthalten. Bevorzugte filmbildende und/oder festigende Polymere sind Copolymere von Maleinsäureanhydrid und Methylvinylether.

Besonders bevorzugte filmbildende und/oder festigende Polymere sind anionisch. Unter einem anionischen Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und keine Struktureinheiten mit permanent kationischen Gruppen aufweist. Unter anionische Gruppen fallen Carboxyl- und Sulfonsäuregruppen.

Die Zusammensetzung einiger illustrativer kosmetischer Mittel kann den folgenden Tabellen entnommen werden (Angaben in Gew.-% bezogen auf das Gesamtgewicht des kosmetischen Mittels sofern nicht anders angegeben). Das Verhältnis der Gewichtsanteile von Copolymer A und Copolymer B in diesen Mitteln liegt zwischen 2:5 und 5:2, vorzugsweise zwischen 1:2 und 2:1 und insbesondere erfindungsgemäß zwischen 2:3 und 3:2.

|  | Formel 1 | Formel 2 | Formel 3 | Formel 4 | Formel 5 |
|---|---|---|---|---|---|
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Alkanolamin *** | 80 bis 100 | 90 bis 100 | 95 bis 100 | 98 | 98 |
| Misc | add 100 | add 100 | add 100 | add 100 | add 100 |
|  | Formel 6 | Formel 7 | Formel 8 | Formel 9 | Formel 10 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| 2-Amino-2-methylpropanol *** | 80 bis 100 | 90 bis 100 | 95 bis 100 | 98 | 98 |
| Misc | add 100 | add 100 | add 100 | add 100 | add 100 |
| * Copolymer A aus den Monomeren a1) n-Butylmethacrylat, a2) Methacrylsäure, a3) Ethylacrylat und a4) Ethylmethacrylat sowie gegebenenfalls weiteren Monomeren | | | | | |
| ** Copolymer B aus den Monomeren b1) N-tert-Octylacrylamid, b2) Acrylsäure und b3) tert.-Butylaminoethylmethacrylsäure sowie gegebenenfalls weiteren Monomeren | | | | | |
| *** % der zur vollständigen Neutralisation der Copolymere A und B benötigten Menge | | | | | |

Die erfindungsgemäßen Mittel enthalten die Polymere in einem kosmetisch akzeptablen Träger. Bevorzugte kosmetisch akzeptable Träger sind wässrige, alkoholische oder wässrigalkoholische Medien. Bevorzugte kosmetisches Mittel enthalten, bezogen auf ihr Gesamtgewicht, einen Wassergehalt von 0 bis 20 Gew.-%, bevorzugt 0,5 bis 20 Gew.-%, vorzugsweise von 1,0 bis 15 Gew.-% und insbesondere von 1,0 bis 10 Gew.-%.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Als zusätzliche Co-Solventien können organische Lösungsmittel oder ein Gemisch aus Lösungsmitteln enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol. Kosmetisches Mittel, die bezogen auf ihr Gesamtgewicht, 20 bis 99, bevorzugt 20 bis 98 Gew.-%, vorzugsweise von 40 bis 90 Gew.-% und insbesondere von 60 bis 85 Gew.-% organisches Lösungsmittel enthalten, werden erfindungsgemäß bevorzugt.

Die Zusammensetzung einiger illustrativer kosmetischer Mittel kann den folgenden Tabellen entnommen werden (Angaben in Gew.-% bezogen auf das Gesamtgewicht des kosmetischen Mittels sofern nicht anders angegeben).

Das Verhältnis der Gewichtsanteile von Copolymer A und Copolymer B in diesen Mitteln liegt zwischen 2:5 und 5:2, vorzugsweise zwischen 1:2 und 2:1 und insbesondere erfindungsgemäß zwischen 2:3 und 3:2.

| | Formel 11 | Formel 12 | Formel 13 | Formel 14 | Formel 15 |
|---|---|---|---|---|---|
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Org. Lösungsmittel | 20 bis 98 | 40 bis 90 | 60 bis 85 | 82 | 82 |
| Misc | add 100 | add 100 | add 100 | add 100 | add 100 |
| | Formel 16 | Formel 17 | Formel 18 | Formel 19 | Formel 20 |
| Copolymer A * | 0,5 bis 10 | 1,0 bis 8,0 | 2,0 bis 8,0 | 8,0 | 3,5 |
| Copolymer B ** | 0,5 bis 10 | 1,0 bis 8,0 | 2,0 bis 8,0 | 3,5 | 8,0 |
| Ethanol | 20 bis 98 | 40 bis 90 | 60 bis 85 | 82 | 82 |
| Misc | add 100 | add 100 | add 100 | add 100 | add 100 |
| * Copolymer A aus den Monomeren a1) n-Butylmethacrylat, a2) Methacrylsäure, a3) Ethylacrylat und a4) Ethylmethacrylat sowie gegebenenfalls weiteren Monomeren<br>** Copolymer B aus den Monomeren b1) N-tert-Octylacrylamid, b2) Acrylsäure und b3) tert.-Butylaminoethylmethacrylsäure sowie gegebenenfalls weiteren Monomeren | | | | | |

Die kosmetischen Mittel weisen bevorzugt einen pH-Wert (25°C) von 3,5 bis 9,0 auf. Besonders bevorzugt ist der pH-Bereich von 4,0 bis und 7,5 und insbesondere von 4,0 bis 6,0. Mittel mit diesen bevorzugten pH-Werten weisen eine besonders gute Hautverträglichkeit auf. Entsprechende Formulierungen eignen sich aufgrund ihres pH-Wertes auch zur Konfektionierung in metallischen Spraydosen, beispielsweise Aerosoldosen.

Die Zusammensetzung einiger illustrativer kosmetischer Mittel kann den folgenden Tabellen entnommen werden (Angaben in Gew.-% bezogen auf das Gesamtgewicht des kosmetischen Mittels sofern nicht anders angegeben). Das Verhältnis der Gewichtsanteile von Copolymer A und Copolymer B in diesen Mitteln liegt zwischen 2:5 und 5:2, vorzugsweise zwischen 1:2 und 2:1 und insbesondere erfindungsgemäß zwischen 2:3 und 3:2.

| | Formel 21 | Formel 22 | Formel 23 | Formel 24 | Formel 25 |
|---|---|---|---|---|---|
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Wasser | 0,5 bis 20 | 1,0 bis 15 | 1,0 bis 10 | 5 | 5 |
| Misc | add 100 | add 100 | add 100 | add 100 | add 100 |
| | Formel 26 | Formel 27 | Formel 28 | Formel 29 | Formel 30 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Wasser | 0,5 bis 20 | 1,0 bis 15 | 1,0 bis 10 | 5 | 5 |
| Misc | add 100 | add 100 | add 100 | add 100 | add 100 |
| pH-Wert | 3,5 bis 9,0 | 4,0 bis 7,5 | 4,0 bis 6,0 | 5,0 | 5,0 |
| * Copolymer A aus den Monomeren a1) n-Butylmethacrylat, a2) Methacrylsäure, a3) Ethylacrylat und a4) Ethylmethacrylat sowie gegebenenfalls weiteren Monomeren<br>** Copolymer B aus den Monomeren b1) N-tert-Octylacrylamid, b2) Acrylsäure und b3) tert.-Butylaminoethylmethacrylsäure sowie gegebenenfalls weiteren Monomeren | | | | | |

Neben den zuvor beschriebenen Copolymeren und Trägersubstanzen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen.

Esteröle, das heißt Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol®868), Cetyloleat, Glycerintricaprylat, Kokosfettalkohol-caprinat/- caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Ein bevorzugtes erfindungsgemäßes kosmetisches Mittel ist dadurch gekennzeichnet, dass das kosmetische Mittel, bezogen auf sein Gesamtgewicht, 0,01 bis 5,0 Gew.-%, vorzugsweise 0,02 bis 4,0 Gew.-% und insbesondere 0,05 bis 2,0 Gew.-% eines Ölkörpers enthält.

Die Zusammensetzung einiger illustrativer kosmetischer Mittel kann den folgenden Tabellen entnommen werden (Angaben in Gew.-% bezogen auf das Gesamtgewicht des kosmetischen Mittels sofern nicht anders angegeben). Das Verhältnis der Gewichtsanteile von Copolymer A und Copolymer B in diesen Mitteln liegt zwischen 2:5 und 5:2, vorzugsweise zwischen 1:2 und 2:1 und insbesondere erfindungsgemäß zwischen 2:3 und 3:2.

| | Formel 31 | Formel 32 | Formel 33 | Formel 34 | Formel 35 |
|---|---|---|---|---|---|
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Ölkörper | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 36 | Formel 37 | Formel 38 | Formel 39 | Formel 40 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Isopropylmyristat | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 41 | Formel 42 | Formel 43 | Formel 44 | Formel 45 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Alkanolamin *** | 80 bis 100 | 90 bis 100 | 95 bis 100 | 98 | 98 |

(fortgesetzt)

| | Formel 41 | Formel 42 | Formel 43 | Formel 44 | Formel 45 |
|---|---|---|---|---|---|
| Isopropylmyristat | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 46 | Formel 47 | Formel 48 | Formel 49 | Formel 50 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| 2-Amino-2-methylpropanol *** | 80 bis 100 | 90 bis 100 | 95 bis 100 | 98 | 98 |
| Ölkörper | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 51 | Formel 52 | Formel 53 | Formel 54 | Formel 55 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Alkanolamin *** | 80 bis 100 | 90 bis 100 | 95 bis 100 | 98 | 98 |
| Org. Lösungsmittel | 20 bis 98 | 40 bis 90 | 60 bis 85 | 82 | 82 |
| Isopropylmyristat | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 56 | Formel 57 | Formel 58 | Formel 59 | Formel 60 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| 2-Amino-2-methylpropanol *** | 80 bis 100 | 90 bis 100 | 95 bis 100 | 98 | 98 |
| Ethanol | 20 bis 98 | 40 bis 90 | 60 bis 85 | 82 | 82 |
| Ölkörper | 0,01 bis 5,0 | 0,02 bis 4,0 | 0,05 bis 2,0 | 0,1 | 0,1 |
| Misc | add 100 | add 100 | add 100 | add 100 | add 100 |
| | Formel 61 | Formel 62 | Formel 63 | Formel 64 | Formel 65 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Alkanolamin *** | 80 bis 100 | 90 bis 100 | 95 bis 100 | 98 | 98 |
| Ethanol | 20 bis 98 | 40 bis 90 | 60 bis 85 | 82 | 82 |
| Ölkörper | 0,01 bis 5,0 | 0,02 bis 4,0 | 0,05 bis 2,0 | 0,1 | 0,1 |
| Wasser | 0,5 bis 20 | 1,0 bis 15 | 1,0 bis 10 | 5 | 5 |
| Misc | add 100 | add 100 | add 100 | add 100 | add 100 |
| | Formel 66 | Formel 67 | Formel 68 | Formel 69 | Formel 70 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| 2-Amino-2-methylpropanol *** | 80 bis 100 | 90 bis 100 | 95 bis 100 | 98 | 98 |
| Org. Lösungsmittel | 20 bis 98 | 40 bis 90 | 60 bis 85 | 82 | 82 |
| Ölkörper | 0,01 bis 5,0 | 0,02 bis 4,0 | 0,05 bis 2,0 | 0,1 | 0,1 |
| Wasser | 0,5 bis 20 | 1,0 bis 15 | 1,0 bis 10 | 5 | 5 |
| Misc | add 100 | add 100 | add 100 | add 100 | add 100 |

(fortgesetzt)

| | Formel 71 | Formel 72 | Formel 73 | Formel 74 | Formel 75 |
|---|---|---|---|---|---|
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| Alkanolamin *** | 80 bis 100 | 90 bis 100 | 95 bis 100 | 98 | 98 |
| Org. Lösungsmittel | 20 bis 98 | 40 bis 90 | 60 bis 85 | 82 | 82 |
| Ölkörper | 0,01 bis 5,0 | 0,02 bis 4,0 | 0,05 bis 2,0 | 0,1 | 0,1 |
| Wasser | 0,5 bis 20 | 1,0 bis 15 | 1,0 bis 10 | 5 | 5 |
| Misc | add 100 | add 100 | add 100 | add 100 | add 100 |
| pH-Wert | 3,5 bis 9,0 | 4,0 bis 7,5 | 4,0 bis 6,0 | 5,0 | 5,0 |
| | Formel 76 | Formel 77 | Formel 78 | Formel 79 | Formel 80 |
| Copolymer A * | 0,5 bis 10 | 1,0 bis 8,0 | 2,0 bis 8,0 | 5,2 | 3,5 |
| Copolymer B ** | 0,5 bis 10 | 1,0 bis 8,0 | 2,0 bis 8,0 | 3,5 | 5,2 |
| 2-Amino-2-methylpropanol *** | 80 bis 100 | 90 bis 100 | 95 bis 100 | 98 | 98 |
| Ethanol | 20 bis 98 | 40 bis 90 | 60 bis 85 | 82 | 82 |
| Isopropylmyristat | 0,01 bis 5,0 | 0,02 bis 4,0 | 0,05 bis 2,0 | 0,1 | 0,1 |
| Wasser | 0,5 bis 20 | 1,0 bis 15 | 1,0 bis 10 | 5 | 5 |
| Misc | add 100 | add 100 | add 100 | add 100 | add 100 |
| pH-Wert | 3,5 bis 9,0 | 4,0 bis 7,5 | 4,0 bis 6,0 | 5,0 | 5,0 |

* Copolymer A aus den Monomeren a1) n-Butylmethacrylat, a2) Methacrylsäure, a3) Ethylacrylat und a4) Ethylmethacrylat ** Copolymer B aus den Monomeren b1) N-tert-Octylacrylamid, b2) Acrylsäure und b3) tert.-Butylaminoethylmethacrylsäure *** % der zur vollständigen Neutralisation der Copolymere A und B benötigten Menge

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Die Formulierung der erfindungsgemäßen Mittel kann in allen für kosmetische Mittel üblichen Formen erfolgen, beispielsweise in Form von Lösungen, die als Haarwasser, Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind. Bevorzugt liegen die erfindungsgemäßen Mittel in flüssiger Form, beispielsweise in Form eines Sprays oder Aerosolsprays vor. In einer alternativen Ausführungsform können dieser Mittel jedoch auch in Gel- oder in Cremeform vorliegen, wobei transparente Gele besonders bevorzugt sind.

Wie weiter oben ausgeführt, enthalten die bevorzugten Aerosolsprays neben weiteren Aktiv- und Hilfsstoffen ein Treibmittel. Erfindungsgemäß geeignete Treibmittel (Treibgase) sind Propan, n-Butan, iso-Butan, Dimethylether (DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Dimethylether als einzigem Treibgas.

Kosmetische Mittel, die das Treibmittel bezogen auf ihr Gesamtgewicht in einer Menge von 10 - 90 Gew.%, bevorzugt 20 - 80 Gew.% und besonders bevorzugt 40 - 70 Gew.-% enthalten, sind erfindungsgemäß bevorzugt. Da den Treibmitteln in Treibmittel-haltigen Zubereitungen auch eine Lösemittelfunktion zukommt, können solche Zubereitungen einen geringen Gehalt an weiteren organischen Lösungsmitteln aufweisen.

Die Zusammensetzung einiger illustrativer Treibmittel-haltiger kosmetischer Mittel kann der folgenden Tabellen entnommen werden.

In dieser Tabelle verweist die linke Spalte ("Formel x") auf jeweils eine der in den weiter oben offenbarten Tabellen angeführten kosmetischen Zusammensetzungen.

Die weiteren Spalten zwei bis fünf ("Treibmittel") geben jeweils die der entsprechenden kosmetischen Zusammensetzung zugesetzte Menge an Treibmittel an. Diese Angaben in "Gew.-%" beziehen sich auf das Gesamtgewicht der kosmetischen Zusammensetzung der jeweiligen "Formel x" ohne Treibmittel.

So beschreiben beispielsweise die Spalten zwei bis fünf in Zeile 12 der nachfolgenden Tabelle vier kosmetische Zubereitungen, umfassend ein kosmetisches Mittel gemäß Formel 11 (siehe entsprechende Tabelle auf Seite 7 oben), umfassend

- 0,5 bis 10 Gew.-% Copolymer A aus den Monomeren a1) n-Butylmethacrylat, a2) Methacrylsäure, a3) Ethylacrylat und a4) Ethylmethacrylat sowie gegebenenfalls weiteren Monomeren;
- 0,5 bis 10 Gew.-% Copolymer B aus den Monomeren b1) N-tert-Octylacrylamid b2) Acrylsäure und b3) tert.-Butylaminoethylmethacrylsäure sowie gegebenenfalls weiteren Monomeren
- 20 bis 98 Gew.-% organisches Lösungsmittel,

welches kosmetische Mittel, bezogen auf sein Gesamtgewicht, mit

- 80 bis 140 Gew.-% Treibmittel (Spalte 2); oder
- 100 Gew.-% Treibmittel (Spalte 3); oder
- 80 bis 140 Gew.-% Dimethylether (DME) (Spalte 4); oder
- 100 Gew.-% Dimethylether (DME) (Spalte 5)

kombiniert wurde.

Eine kosmetische Zubereitung gemäß Zeile 12, Spalte 5 der nachfolgenden Tabelle, umfasst mit anderen Worten eine 1:1 Mischung des Treibmittel-freien kosmetischen Mittels gemäß Formel 11 mit Dimethylether.

| | Treibmittel [Gew.-%] | | | |
|---|---|---|---|---|
| Formel 1 | 80 bis 140 | 100 | 80 bis 140 DME | 100 DME |
| Formel 2 | 80 bis 140 | 100 | 80 bis 140 DME | 100 DME |
| Formel 3 | 80 bis 140 | 100 | 80 bis 140 DME | 100 DME |
| Formel 4 | 80 bis 140 | 100 | 80 bis 140 DME | 100 DME |
| Formel 5 | 80 bis 140 | 100 | 80 bis 140 DME | 100 DME |
| Formel 6 | 80 bis 140 | 100 | 80 bis 140 DME | 100 DME |
| Formel 7 | 80 bis 140 | 100 | 80 bis 140 DME | 100 DME |
| Formel 8 | 80 bis 140 | 100 | 80 bis 140 DME | 100 DME |
| Formel 9 | 80 bis 140 | 100 | 80 bis 140 DME | 100 DME |
| Formel 10 | 80 bis 140 | 100 | 80 bis 140 DME | 100 DME |
| Formel 11 | 80 bis 140 | 100 | 80 bis 140 DME | 100 DME |
| Formel 12 | 80 bis 140 | 100 | 80 bis 140 DME | 100 DME |
| Formel 13 | 80 bis 140 | 100 | 80 bis 140 DME | 100 DME |
| Formel 14 | 80 bis 140 | 100 | 80 bis 140 DME | 100 DME |
| Formel 15 | 80 bis 140 | 100 | 80 bis 140 DME | 100 DME |
| Formel 16 | 80 bis 140 | 100 | 80 bis 140 DME | 100 DME |
| Formel 17 | 80 bis 140 | 100 | 80 bis 140 DME | 100 DME |
| Formel 18 | 80 bis 140 | 100 | 80 bis 140 DME | 100 DME |
| Formel 19 | 80 bis 140 | 100 | 80 bis 140 DME | 100 DME |
| Formel 20 | 80 bis 140 | 100 | 80 bis 140 DME | 100 DME |

(fortgesetzt)

|  | Treibmittel [Gew.-%] | | | |
|---|---|---|---|---|
| Formel 21 | 80 bis 140 | 100 | 80 bis 140 DME | 100 DME |
| Formel 22 | 80 bis 140 | 100 | 80 bis 140 DME | 100 DME |
| Formel 23 | 80 bis 140 | 100 | 80 bis 140 DME | 100 DME |
| Formel 24 | 80 bis 140 | 100 | 80 bis 140 DME | 100 DME |
| Formel 25 | 80 bis 140 | 100 | 80 bis 140 DME | 100 DME |
| Formel 26 | 80 bis 140 | 100 | 80 bis 140 DME | 100 DME |
| Formel 27 | 80 bis 140 | 100 | 80 bis 140 DME | 100 DME |
| Formel 28 | 80 bis 140 | 100 | 80 bis 140 DME | 100 DME |
| Formel 29 | 80 bis 140 | 100 | 80 bis 140 DME | 100 DME |
| Formel 30 | 80 bis 140 | 100 | 80 bis 140 DME | 100 DME |
| Formel 31 | 80 bis 140 | 100 | 80 bis 140 DME | 100 DME |
| Formel 32 | 80 bis 140 | 100 | 80 bis 140 DME | 100 DME |
| Formel 33 | 80 bis 140 | 100 | 80 bis 140 DME | 100 DME |
| Formel 34 | 80 bis 140 | 100 | 80 bis 140 DME | 100 DME |
| Formel 35 | 80 bis 140 | 100 | 80 bis 140 DME | 100 DME |
| Formel 36 | 80 bis 140 | 100 | 80 bis 140 DME | 100 DME |
| Formel 37 | 80 bis 140 | 100 | 80 bis 140 DME | 100 DME |
| Formel 38 | 80 bis 140 | 100 | 80 bis 140 DME | 100 DME |
| Formel 39 | 80 bis 140 | 100 | 80 bis 140 DME | 100 DME |
| Formel 40 | 80 bis 140 | 100 | 80 bis 140 DME | 100 DME |
| Formel 41 | 80 bis 140 | 100 | 80 bis 140 DME | 100 DME |
| Formel 42 | 80 bis 140 | 100 | 80 bis 140 DME | 100 DME |
| Formel 43 | 80 bis 140 | 100 | 80 bis 140 DME | 100 DME |
| Formel 44 | 80 bis 140 | 100 | 80 bis 140 DME | 100 DME |
| Formel 45 | 80 bis 140 | 100 | 80 bis 140 DME | 100 DME |
| Formel 46 | 80 bis 140 | 100 | 80 bis 140 DME | 100 DME |
| Formel 47 | 80 bis 140 | 100 | 80 bis 140 DME | 100 DME |
| Formel 48 | 80 bis 140 | 100 | 80 bis 140 DME | 100 DME |
| Formel 49 | 80 bis 140 | 100 | 80 bis 140 DME | 100 DME |
| Formel 50 | 80 bis 140 | 100 | 80 bis 140 DME | 100 DME |
| Formel 51 | 80 bis 140 | 100 | 80 bis 140 DME | 100 DME |
| Formel 52 | 80 bis 140 | 100 | 80 bis 140 DME | 100 DME |
| Formel 53 | 80 bis 140 | 100 | 80 bis 140 DME | 100 DME |
| Formel 54 | 80 bis 140 | 100 | 80 bis 140 DME | 100 DME |
| Formel 55 | 80 bis 140 | 100 | 80 bis 140 DME | 100 DME |
| Formel 56 | 80 bis 140 | 100 | 80 bis 140 DME | 100 DME |
| Formel 57 | 80 bis 140 | 100 | 80 bis 140 DME | 100 DME |
| Formel 58 | 80 bis 140 | 100 | 80 bis 140 DME | 100 DME |

(fortgesetzt)

| | Treibmittel [Gew.-%] | | | |
|---|---|---|---|---|
| Formel 59 | 80 bis 140 | 100 | 80 bis 140 DME | 100 DME |
| Formel 60 | 80 bis 140 | 100 | 80 bis 140 DME | 100 DME |
| Formel 61 | 80 bis 140 | 100 | 80 bis 140 DME | 100 DME |
| Formel 62 | 80 bis 140 | 100 | 80 bis 140 DME | 100 DME |
| Formel 63 | 80 bis 140 | 100 | 80 bis 140 DME | 100 DME |
| Formel 64 | 80 bis 140 | 100 | 80 bis 140 DME | 100 DME |
| Formel 65 | 80 bis 140 | 100 | 80 bis 140 DME | 100 DME |
| Formel 66 | 80 bis 140 | 100 | 80 bis 140 DME | 100 DME |
| Formel 67 | 80 bis 140 | 100 | 80 bis 140 DME | 100 DME |
| Formel 68 | 80 bis 140 | 100 | 80 bis 140 DME | 100 DME |
| Formel 69 | 80 bis 140 | 100 | 80 bis 140 DME | 100 DME |
| Formel 70 | 80 bis 140 | 100 | 80 bis 140 DME | 100 DME |
| Formel 71 | 80 bis 140 | 100 | 80 bis 140 DME | 100 DME |
| Formel 72 | 80 bis 140 | 100 | 80 bis 140 DME | 100 DME |
| Formel 73 | 80 bis 140 | 100 | 80 bis 140 DME | 100 DME |
| Formel 74 | 80 bis 140 | 100 | 80 bis 140 DME | 100 DME |
| Formel 75 | 80 bis 140 | 100 | 80 bis 140 DME | 100 DME |
| Formel 76 | 80 bis 140 | 100 | 80 bis 140 DME | 100 DME |
| Formel 77 | 80 bis 140 | 100 | 80 bis 140 DME | 100 DME |
| Formel 78 | 80 bis 140 | 100 | 80 bis 140 DME | 100 DME |
| Formel 79 | 80 bis 140 | 100 | 80 bis 140 DME | 100 DME |
| Formel 80 | 80 bis 140 | 100 | 80 bis 140 DME | 100 DME |

Die Verwendung eines erfindungsgemäßen kosmetischen Mittels zur temporären Verformung keratinischer Fasern ist ein weiterer Gegenstand der vorliegenden Anmeldung. Wie eingangs ausgeführt, zeichnen sich die erfindungsgemäßen Mittel insbesondere durch einen verbesserten Halt bei der temporären Verformung keratinischer Fasern aus. Ein zusätzlicher Gegenstand der vorliegenden Anmeldung ist daher die Verwendung eines erfindungsgemäßen kosmetischen Mittels zur Verbesserung des Halts bei der temporären Verformung keratinischer Fasern.

Beispiele

Folgende Zusammensetzungen wurden durch Vermischen der angegebenen Rohstoffe bereitgestellt (Die Mengenangaben verstehen sich - soweit nichts anderes vermerkt ist - in Gewichtsprozent.):

| | 1 | 2 | 3 | 4 | 5 |
|---|---|---|---|---|---|
| Copolymer A * | - | 1 | 2 | 3 | 4 |
| Copolymer B ** | 4 | 3 | 2 | 1 | - |
| Ethanol | 96 | 96 | 96 | 96 | 96 |
| * Copolymer A aus den Monomeren a1) n-Butylmethacrylat, a2) Methacrylsäure, a3) Ethylacrylat und a4) Ethylmethacrylat<br>** Copolymer B aus den Monomeren b1) N-tert-Octylacrylamid, b2) Acrylsäure und b3) tert.-Butylaminoethylmethacrylsäure | | | | | |

Die Zusammensetzung 3 ist erfindungsgemäß, die Zusammensetzungen 1, 2, 4 und 5 dienen Vergleichszwecken.

Diese Zusammensetzungen wurden mittels einer High Humidity Curl Retention Messung hinsichtlich ihrer formgebenden Eigenschaften überprüft.

Hierzu wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0,6 g eingesetzt. Zur Vorbereitung wurden die Strähnen mit einer 12,5 Gew.-%igen Natriumlaurethsulphat-Lösung gewaschen. Die Haarsträhnen wurden über Nacht in einem Trockenofen bei 318 K getrocknet.

Jeweils 0,18 G der Zusammensetzungen 1 bis 5 wurden auf eine Haarsträhne appliziert und einmassiert. Die Strähne wurden dann auf einen Wickler gewickelt (Fripac-medis, Durchmesser 7 mm, Art. Nr. D-1203) und über Nacht bei Raumtemperatur getrocknet.

Die Wickler wurden vorsichtig entfernt und die Strähnen aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85 % über einen Zeitraum von 24 H gelagert. Anschließend wurden die Längen der Locken vermessen ($L_t$).

Für jede der Zusammensetzungen 1 bis 5 wurden fünf Teststrähnen vermessen.

Die High-Humidity Curl-Retention (HHCR) wurde nach folgender Formel berechnet:

$$HHCR = (L_{max} - L_t) / (L_{max} - L_0)$$

Für jede Zusammensetzung wurde das arithmetische Mittel aus den HHCR-Werten der fünf Teststrähnen gebildet.

Die Ergebnisse dieser Messungen finden sich in der nachfolgenden Tabelle:

|      | 1    | 2    | 3    | 4    | 5    |
|------|------|------|------|------|------|
| HHCR | 0,70 | 0,71 | 0,75 | 0,72 | 0,71 |

**Patentansprüche**

1. Kosmetisches Mittel, enthaltend in einem kosmetisch akzeptablen Träger

   a) mindestens ein Copolymer A aus den Monomeren

   a1) n-Butylmethacrylat
   a2) Methacrylsäure
   a3) Ethylacrylat
   a4) Ethylmethacrylat

   sowie gegebenenfalls weiteren Monomeren
   b) mindestens ein von Copolymer A unterschiedliches Copolymer B aus den Monomeren

   b1) N-tert-Octylacrylamid
   b2) Acrylsäure
   b3) tert.-Butylaminoethylmethacrylsäure
   sowie gegebenenfalls weiteren Monomeren,

   **dadurch gekennzeichnet, dass** das Verhältnis der Gewichtsanteile von Copolymer A und Copolymer B in dem Mittel zwischen 2:3 und 3:2 beträgt.

2. Kosmetisches Mittel nach Anspruch 1, **dadurch gekennzeichnet, dass** der Gewichtsanteil des Copolymers A am Gesamtgewicht des kosmetischen Mittels zwischen 0,5 und 10 Gew.-%, vorzugsweise zwischen 1,0 und 8,0 Gew.-% und insbesondere zwischen 2,0 und 8,0 Gew.-% beträgt.

3. Kosmetisches Mittel nach einem der vorgehenden Ansprüche, **dadurch gekennzeichnet, dass** der Gewichtsanteil des Copolymers B am Gesamtgewicht des kosmetischen Mittels zwischen 0,5 und 10 Gew.-%, vorzugsweise zwischen 1,0 und 8,0 Gew.-% und insbesondere zwischen 2,0 und 8,0 Gew.-% beträgt.

**4.** Kosmetisches Mittel nach einem der vorgehenden Ansprüche, **dadurch gekennzeichnet, dass** das kosmetische Mittel 2-Amino-2-methylpropanol enthält, wobei der Gewichtsanteil des 2-Amino-2-methylpropanols am Gesamtgewicht des kosmetischen Mittels vorzugsweise 0,1 bis 4,0 Gew.-%, bevorzugt 0,2 bis 3,0 Gew.% und insbesondere 0,5 bis 2,0 Gew.-% beträgt.

**5.** Kosmetisches Mittel nach einem der vorgehenden Ansprüche, **dadurch gekennzeichnet, dass** das kosmetische Mittel, bezogen auf sein Gesamtgewicht, 0,01 bis 5,0 Gew.-%, vorzugsweise 0,02 bis 4,0 Gew.-% und insbesondere 0,05 bis 2,0 Gew.-% eines Fettstoffs enthält.

**6.** Kosmetisches Mittel nach einem der vorgehenden Ansprüche, **dadurch gekennzeichnet, dass** das kosmetische Mittel, bezogen auf sein Gesamtgewicht, 20 bis 98 Gew.-%, vorzugsweise von 40 bis 90 Gew.-% und insbesondere von 60 bis 85 Gew.-% organisches Lösungsmittel enthält.

**7.** Kosmetisches Mittel nach einem der vorgehenden Ansprüche, **dadurch gekennzeichnet, dass** das kosmetische Mittel bezogen auf sein Gesamtgewicht einen Wassergehalt von 0,5 bis 20 Gew.-%, vorzugsweise von 1,0 bis 15 Gew.-% und insbesondere von 1,0 bis 10 Gew.-% aufweist.

**8.** Kosmetisches Mittel nach einem der vorgehenden Ansprüche, **dadurch gekennzeichnet, dass** das kosmetische Mittel einen pH-Wert (25°C) von 3,5 bis 9,0, vorzugsweise von 4,0 bis 7,5 und insbesondere von 4,0 bis 6,0 auf weist.

**9.** Verwendung eines kosmetischen Mittels nach einem der Ansprüche 1 bis 8 zur temporären Verformung keratinischer Fasern.

**Claims**

**1.** A cosmetic agent containing

a) at least one copolymer A consisting of the monomers

a1) n-butyl methacrylate
a2) methacrylic acid
a3) ethyl acrylate
a4) ethyl methacrylate

and possibly other monomers, and
b) at least one copolymer B, which is different from copolymer A, consisting of the monomers

b1) N-tert-octylacrylamide
b2) acrylic acid
b3) tert-butylaminoethyl methacrylic acid

and possibly other monomers,

in a cosmetically acceptable carrier, **characterized in that** the ratio of the percentages by weight of copolymer A and copolymer B in the agent is between 2:3 and 3:2.

**2.** The cosmetic agent according to claim 1, **characterized in that** the percentage by weight of copolymer A is between 0.5 and 10 wt.%, preferably between 1.0 and 8.0 wt.%, and in particular between 2.0 and 8.0 wt.% of the overall weight of the cosmetic agent.

**3.** The cosmetic agent according to one of the preceding claims, **characterized in that** the percentage by weight of copolymer B is between 0.5 and 10 wt.%, preferably between 1.0 and 8.0 wt.%, and in particular between 2.0 and 8.0 wt.% of the overall weight of the cosmetic agent.

**4.** The cosmetic agent according to one of the preceding claims, **characterized in that** the cosmetic agent contains 2-amino-2-methylpropanol, the percentage by weight of the 2-amino-2-methyl propanol preferably being between 0.1 and 4.0 wt.%, preferably 0.2 and 3.0 wt.%, and in particular 0.5 and 2.0 wt.% of the overall weight of the cosmetic agent.

**5.** The cosmetic agent according to one of the preceding claims, **characterized in that** the cosmetic agent contains from 0.01 to 5.0 wt.%, preferably from 0.02 to 4.0 wt.%, and in particular from 0.05 to 2.0 wt.% of a fatty substance, based on its overall weight.

**6.** The cosmetic agent according to one of the preceding claims, **characterized in that** the cosmetic agent contains from 20 to 98 wt.%, preferably from 40 to 90 wt.%, and in particular from 60 to 85 wt.% organic solvent, based on its overall weight.

**7.** The cosmetic agent according to one of the preceding claims, **characterized in that** the cosmetic agent has a water content of from 0.5 to 20 wt.%, preferably from 1.0 to 15 wt.%, and in particular from 1.0 to 10 wt.%, based on its overall weight.

**8.** The cosmetic agent according to one of the preceding claims, **characterized in that** the cosmetic agent has a pH (25 °C) of from 3.5 to 9.0, preferably from 4.0 to 7.5 and in particular from 4.0 to 6.0.

**9.** The use of a cosmetic agent according to one of claims 1 to 8 for temporarily deforming keratin fibers.

**Revendications**

**1.** Cosmétique contenant un vecteur cosmétiquement acceptable :

a) au moins un copolymère A issu des monomères

a1) méthacrylate de n-butyle
a2) acide méthacrylique
a3) acrylate d'éthyle
a4) méthacrylate d'éthyle

et éventuellement d'autres monomères
b) au moins un copolymère B différent du copolymère A parmi les monomères :

b1) N-tert-octylacrylamide
b2) acide acrylique
b3) acide tert-butylaminoéthylméthacrylique,

**caractérisé en ce que** le rapport pondéral entre le copolymère A et la copolymère B dans le produit se situe entre 2:3 et 3:2.

**2.** Produit cosmétique selon la revendication 1, **caractérisé en ce que** le rapport pondéral du copolymère A au poids total du produit cosmétique se situe entre 0,5 et 10 % en poids, de préférence entre 1,0 et 8,0 % en poids, et en particulier entre 2,0 et 8,0 % en poids.

**3.** Produit cosmétique une des revendications précédentes, **caractérisé en ce que** le rapport pondéral du copolymère B au poids total du produit cosmétique se situe entre 0,5 et 10 % en poids, de préférence entre 1,0 et 8,0 % en poids, et en particulier entre 2,0 et 8,0 % en poids.

**4.** Produit cosmétique une des revendications précédentes, **caractérisé en ce que** le produit cosmétique contient du 2-amino-2-méthylpropanol, le rapport pondéral du 2-amino-2-méthylpropanol sur le poids total du produit cosmétique étant préférentiellement entre 0,1 et 4,0 % en poids, de préférence entre 0,2 et 3,0 % en poids, et en particulier entre 0,5 et 2,0 % en poids.

**5.** Produit cosmétique une des revendications précédentes, **caractérisé en ce que** le produit cosmétique contient une graisse à hauteur d'entre 0,01 et 5,0 % en poids rapporté à son poids total, de préférence entre 0,02 et 4,0 % en poids, et en particulier entre 0,05 et 2,0 % en poids.

**6.** Produit cosmétique une des revendications précédentes, **caractérisé en ce que** le produit cosmétique contient un solvant organique à hauteur d'entre 20 et 98 % en poids rapporté à son poids total, de préférence entre 40 et 90 % en poids, et en particulier entre 60 et 85 % en poids.

**7.** Produit cosmétique une des revendications précédentes, **caractérisé en ce que** le produit cosmétique présente une teneur en eau entre 0,5 et 20 % en poids rapporté à son poids total, de préférence entre 1,0 et 15 % en poids, et en particulier entre 1,0 et 10 % en poids.

**8.** Produit cosmétique une des revendications précédentes, **caractérisé en ce que** le produit cosmétique présente un pH (à 25°C) entre 3,5 et 9,0, de préférence entre 4,0 et 7,5, et en particulier entre 4,0 et 6,0.

**9.** Utilisation d'un produit cosmétique selon une des revendications 1 à 8 pour la mise en forme temporaire de fibres de kératine.

**EP 2 779 988 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011057882 A1